# EUROPEAN PATENT SPECIFICATION

(11) **EP 4 044 994 B1**
(45) Date of publication and mention of the grant of the patent: **14.06.2023**
(21) Application number: 20789095.5
(22) Date of filing: 08.10.2020
(51) Int. Cl.: A61K 8/27, A61Q 5/00, A61Q 5/02, A61K 8/46

(54) **HAIR TREATMENT METHOD**
HAARBEHANDLUNGSVERFAHREN
PROCÉDÉ DE TRAITEMENT CAPILLAIRE

(30) Priority: 18.10.2019 EP 19204138
(43) Date of publication of application: 24.08.2022
(73) Proprietor: Unilever IP Holdings B.V., 3013 AL Rotterdam (NL); Unilever Global IP Limited, Wirral, Merseyside CH62 4ZD (GB)
(72) Inventor: ZHOU, Rongrong, Wirral Merseyside CH63 3JW (GB)
(74) Representative: Chisem, Janet
(86) International application number: PCT/EP2020/078332
(87) International publication number: WO 2021/074012

(56) References cited:
- WO-A1-2016/024599
- CN-A- 109 260 038
- DE-A1- 4 225 985
- JP-A- 2019 104 706
- US-A1- 2016 095 802
- R Kon ET AL: "Analysis of the damaged components of permed hair using biochemical technique", Journal of Cosmetic Science, 28 February 1998 (1998-02-28), pages 13-22, XP055684895, Retrieved from the Internet: URL:http://citeseerx.ist.psu.edu/viewdoc/d ownload?doi=10.1.1.574.6064&rep=rep1&type= pdf [retrieved on 2020-04-09]
- TAKAFUMI INOUE ET AL: "Labile proteins accumulated in damaged hair upon permanent waving and bleaching treatments", JOURNAL OF COSMETIC SCIENCE, vol. 53, no. 6, 1 November 2002 (2002-11-01), pages 337-344, XP055345049, US ISSN: 1525-7886
- R. SINCLAIR ET AL: "The proteomic profile of hair damage", BRITISH JOURNAL OF DERMATOLOGY, vol. 166, 7 June 2012 (2012-06-07), pages 27-32, XP055201716, ISSN: 0007-0963, DOI: 10.1111/j.1365-2133.2012.10862.x

## Description

### Field of the Invention

The present invention relates to a method of repairing damaged hair using compositions comprising zinc compounds.

### Background and Prior Art

Consumers regularly subject their hair to intensive treatment, and care and styling routines to help them achieve their desired look. The actions performed by consumers introduce modifications to the chemistry of hair keratin protein which results in micro- and macro-structural changes and, in turn, changes fibre physical properties: the consequences of these are generally perceived by the consumer as damage.

Combing and brushing of hair mechanically abrades the fibre cuticle making this rougher and increasing the frictional characteristics. Hair lightening, such as bleaching, or colouring treatments generally involve an oxidative step to break down melanin and develop the new hair colour, but these processes also oxidise the hair fibre protein and the endogenous lipids. These reactions alter the number and types of covalent and noncovalent bonds within the fibre and impact the thermal stability and mechanical properties of the hair. The internal protein of damaged hair typically has a reduced denaturation temperature compared to that of virgin hair.

US 4 960 588 discloses metal salts having a valency of at least II in hair styling compositions to improve set retention.

WO 2012/156177 discloses a method of mitigating hair damage comprising the step of applying to the hair a composition comprising a group III metal salt; aluminium being preferred and exemplified.

US2017/360674 discloses a cosmetic agent for treating keratin fibres that comprises an acid protein and at least one salt comprising at least one divalent or trivalent cation, and a method for maintaining the colour of dyed keratin fibres using the cosmetic agent.

WO2012/084866 and WO2012/084903 are concerned with protection of artificial colour of hair, prevention of fading over time and giving hair good cosmetic properties. The former disclosed a cosmetic composition comprising one or more non-nitrogenous zinc salts and one or more quaternary ammonium salts in a weight ratio of quat to zinc salt of less than or equal to 1; the latter, a cosmetic composition comprising one or more non-nitrogenous zinc salts and one or more amino silicones in a weight ratio of silicone to zinc element of 0.01 to 5.

Despite the prior art there remains a need for new effective treatments for damaged hair.

The present inventors have found that hair treated with compositions comprising certain zinc compounds can repair damaged hair.

### Definition of the Invention

A first aspect of the invention provides a method of repairing damaged internal protein of hair by increasing the denaturation temperature of the internal protein, comprising the step of applying a shampoo composition to the hair, wherein the composition comprises a soluble zinc compound selected from zinc sulphate, zinc chloride, zinc gluconate, zinc acetate, zinc citrate and mixtures thereof and a cleansing surfactant selected from anionic surfactant and mixtures thereof; and wherein the composition has a pH of from 3 to 5, and leaving the composition on the hair for a maximum time period of 20 minutes and rinsing the composition from the hair.

A use is also provided, of a composition as defined above, to repair damage to internal hair protein.

The method of the invention provides damage repair to internal hair protein by increasing the denaturation temperature of the internal protein of hair.

The cause of the damage may be selected from mechanical means, chemical means and environmental means. Preferably, the damage is selected from lightening, chemical straightening, colouring, heat styling, and mixtures thereof.

The method comprises the step of rinsing the composition from the hair.

### Zinc Compounds

Compositions for use in the invention comprise a soluble zinc compound.

By soluble is meant a solubility of 4.0 × 10⁴ mg/L or more in water at 25°C.

The zinc compounds are selected from zinc sulphate, zinc chloride, zinc gluconate, zinc acetate, zinc citrate and mixtures thereof, most preferably zinc sulphate.

The level of zinc compound in the total composition is preferably from 0.005 to 10 wt%, more preferably from 0.01 to 5 wt%, most preferably from 0.05 to 2 wt%.

The pH of the formulations of the invention are in the range from pH 3 to pH 6, more preferably used at pH 3-5.

Hair treatment compositions for use in the invention take the form of shampoos

The composition is formulated as a rinse off product. In the context of this invention rinse off products are applied to the hair left for a maximum time of 20 minutes then rinsed off.

The method of treatment comprises the following sequential steps
i) application of a composition comprising a zinc compound selected from zinc sulphate, zinc chloride, zinc gluconate, zinc acetate, zinc citrate and mixtures thereof, a cleansing surfactant selected from anionic surfactant and mixtures thereof and having a pH of 3 - 5 for a maximum time period of 20 minutes, more preferably 5 minutes, most preferably 30 seconds;
ii) rinsing the hair.

Shampoo compositions for use in method of the invention are generally aqueous, i.e. they have water or an aqueous solution or a lyotropic liquid crystalline phase as their major component.

Suitably, the shampoo composition will comprise from 50 to 98%, preferably from 60 to 90% water by weight based on the total weight of the composition.

Shampoo compositions will comprise one or more cleansing surfactants.

Surfactants are compounds which have hydrophilic and hydrophobic portions that act to reduce the surface tension of the aqueous solutions they are dissolved in. Shampoo compositions for use in the method of the invention will generally comprise one or more cleansing surfactants, which are cosmetically acceptable and suitable for topical application to the hair. The shampoo composition comprises a cleansing surfactant selected from anionic surfactants.

The total amount of cleansing surfactant in a shampoo composition for use in the invention is generally from 1 to 50%, preferably from 2 to 40%, more preferably from 4 to 25% by total weight surfactant based on the total weight of the composition.

Non-limiting examples cleansing surfactants include anionic cleansing surfactants including alkyl sulphates, alkyl ether sulphates, alkaryl sulphonates, N-alkyl sarcosinates, alkyl phosphates, alkyl ether phosphates, acyl amino acid based surfactants, alkyl ether carboxylic acids, acyl taurates, acyl glutamates, alkyl glycinates and salts thereof, especially their sodium, magnesium, ammonium and mono-, di- and triethanolamine salts. The alkyl and acyl groups in the preceding list generally contain from 8 to 18, preferably from 10 to 16 carbon atoms and may be unsaturated. The alkyl ether sulphates, alkyl ether phosphates and alkyl ether carboxylic acids and salts thereof may contain from 1 to 20 ethylene oxide or propylene oxide units per molecule.

Further non-limiting examples of cleansing surfactants may include non-ionic cleansing surfactants including; aliphatic (C₈ - C₁₈) primary or secondary linear or branched chain alcohols with alkylene oxides, usually ethylene oxide and generally having from 6 to 30 ethylene oxide groups. Other representative cleansing surfactants include mono- or di-alkyl alkanolamides (examples include coco mono-ethanolamide and coco monoisopropanolamide) and alkyl polyglycosides (APGs). Suitable alkyl polyglycosides for use in the invention are commercially available and include for example those materials identified as: Plantapon 1200 and Plantapon 2000 ex BASF. Other sugar-derived surfactants, which can be included in compositions for use in the invention include the C₁₀-C₁₈ N-alkyl (C_{I}-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

Additional non-limiting examples of cleansing surfactants may include amphoteric or zwitterionic cleansing surfactants including; alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms.

Typical cleansing surfactants for use in shampoo compositions for use in the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium cocoyl isethionate, sodium lauryl isethionate, lauryl ether carboxylic acid and sodium N-lauryl sarcosinate, sodium pareth sulphate, cocodimethyl sulphopropyl betaine, lauryl betaine, coco betaine, cocamidopropyl betaine, sodium cocoamphoacetate.

Preferred cleansing surfactants are sodium lauryl sulphate, sodium lauryl ether sulphate (n)EO, (where n is from 1 to 3, preferably 2 to 3, most preferably 3), ammonium lauryl sulphate, ammonium lauryl ether sulphate(n)EO, (where n is from 1 to 3, preferably 2 to 3, most preferably 3), sodium cocoyl isethionate and lauryl ether carboxylic acid, coco betaine, cocamidopropyl betaine, sodium cocoamphoacetate.

Mixtures of any of the foregoing anionic, non-ionic and amphoteric cleansing surfactants may also be suitable, preferably where the primary to secondary surfactant ratio is between 1:1 - 10:1, more preferably 2:1 - 9:1 and most preferably 3:1 - 8:1, based on the inclusion weight of the cleansing surfactant in the shampoo composition.

Optionally, a shampoo composition for use in the invention may contain further ingredients, (non-limiting examples of which are described below) to enhance performance and/or consumer acceptability.

Cationic polymers are preferred ingredients in a shampoo composition for use in the invention for enhancing conditioning performance.

Suitable cationic polymers may be homopolymers which are cationically substituted or may be formed from two or more types of monomers. The weight average (M_{w}) molecular weight of the polymers will generally be between 100 000 and 3 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general, secondary and tertiary amines, especially tertiary, are preferred.

Amine substituted vinyl monomers and amines can be polymerised in the amine form and then converted to ammonium by quaternization.

The cationic polymers can comprise mixtures of monomer units derived from amine- and/or quaternary ammonium-substituted monomer and/or compatible spacer monomers.

Suitable (non-limiting examples of) cationic polymers include:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions for use in the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581). Examples of such materials include the polymer LR and JR series from Dow, generally referred to in the industry (CTFA) as Polyquaternium 10.

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14 and JAGUAR C17.

Mixtures of any of the above cationic polymers may be used.

Cationic polymer will generally be present in a shampoo composition for use in the invention at levels of from 0.01 to 5%, preferably from 0.02 to 1%, more preferably from 0.05 to 0.8% by total weight of cationic polymer based on the total weight of the composition.

Preferably a composition for use in the method of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition for use in the method of the invention at levels of from 0.1 to 10%, preferably from 0.5 to 6%, more preferably from 0.9 to 4% by total weight of suspending agent based on the total weight of the composition.

Compositions for use in the method of the invention will preferably also contain one or more silicone conditioning agents, particularly emulsified droplets of a silicone conditioning agent, for enhancing conditioning performance.

The emulsified silicone is preferably selected from the group consisting of polydiorganosiloxanes, silicone gums, amino functional silicones and mixtures thereof.

Suitable silicones include polydiorganosiloxanes, in particular polydimethylsiloxanes which have the CTFA designation dimethicone. Also suitable for use in compositions for use in the method of the invention (particularly shampoos and conditioners) are polydimethyl siloxanes having hydroxyl end groups, which have the CTFA designation dimethiconol. Also suitable are silicone gums having a slight degree of cross-linking, as are described for example in WO 96/31188.

The viscosity of the emulsified silicone itself (not the emulsion or the final hair conditioning composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the shampoo compositions will typically have a D90 silicone droplet size in the composition of less than 30, preferably less than 20, more preferably less than 10 micron, ideally from 0.01 to 1 micron. Silicone emulsions having an average silicone droplet size (D50) of 0.15 micron are generally termed microemulsions.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

The total amount of silicone is preferably from 0.01 wt% to 10 %wt of the total composition more preferably from 0.1 wt% to 5 wt%, most preferably 0.5 wt% to 3 wt% is a suitable level.

A composition for use in the method of the invention may contain other ingredients for enhancing performance and/or consumer acceptability. Such ingredients include fragrance, dyes and pigments, pH adjusting agents, pearlescers or opacifiers, viscosity modifiers, and preservatives or antimicrobials. Each of these ingredients will be present in an amount effective to accomplish its purpose. Generally these optional ingredients are included individually at a level of up to 5% by weight of the total composition.

Hair treatment compositions for use in the method and use of the invention are primarily intended for topical application to the hair and/or scalp of a human subject, for the treatment of, damaged hair. They are rinse-off compositions.

Similarly, the use pertaining to the second aspect of the invention, involves topical application of a soluble zinc compound to hair.

The invention will be further illustrated by the following, non-limiting Examples, in which all percentages quoted are by weight based on total weight unless otherwise stated.

### EXAMPLES

### The Hair

Virgin: The hair used in the following examples was dark brown European hair tresses 2 grams and 10 inches long.

Bleached: Virgin hair tresses were bleached according to the following protocol. Hair was bleached for 30 minutes with commercial bleaching powder mixed with 9% cream peroxide, 30 'vol'. Hair was then washed with 14% aqueous sodium laureth ether sulphate (SLES) solution before drying.

The double-bleached hair was dialysed prior to the experiments in 5L of distilled water over a period of 72 hours, and the water was changed 3 times over this period. After dialysing, the hair tresses were left to dry overnight in a controlled environment (20°C and 50% relative humidity).

### The Compositions

Composition 1, in accordance with the invention, is a shampoo composition. Compositions A is a Comparative shampoo that does not contain any damage repair agents.

The compositions 1 and A are given in Table 1 below.

**Table 1: Ingredients (wt%) of Composition 1 in accordance with the invention and comparative composition A.**

| Ingredient | Amount (wt %) | |
|---|---|---|
| | 1 | A |
| Zinc Sulphate heptahydrate | 1.5 | - |
| Suspending agent | 0.3 | 0.3 |
| Sodium Laureth Ether Sulfate | 14.0 | 14.0 |
| Co-surfactant | 1.6 | 1.6 |
| Deposition aid | 0.2 | 0.2 |
| Silicone | 3 | 3 |
| Sodium Chloride | 0.55 | 0.55 |
| Perfume | 0.55 | 0.55 |
| Water & minors | to 100 | to 100 |
| pH | 4.8 | 6.0 |

### Treatment of the Hair

Hair (virgin and double-bleached) was first treated twice with an aqueous composition containing 14% Sodium Laureth Ether Sulphate (SLES) at 0.1 ml/1g hair using 30 seconds lathering and 30 seconds rinse in tap water.

The hair was then treated with shampoo Composition 1 or A using the following method: 0.1 ml/1g hair applied and lathered for 30 seconds followed by 30 seconds rinse in tap water. After removing excess water, hair treatment was repeated. The hair tresses were then left to dry overnight at 20°C, 50% relative humidity.

### Effect of Treatment by Composition 1 and Comparative Composition A on hair protein

In order to prepare hair samples for Differential Scanning Calorimetry (DSC), 1 inch of hair was cut from the tip-end of each tress. Hair was then chopped into 1-2 mm sections.

Measurements were performed using a Mettler-Toledo DSC (with auto-sampler). 7-10 mg samples of dry, finely chopped hair was placed in the 'Medium Pressure Stainless Steel DSC Pans' and accurately weighed. 50 microlitres of deionised water was then added to each sample after which the pan lid was put on and the pans crimped shut to provide a hermetic seal. Pans were equilibrated for a minimum of 24 h ahead of any measurement to allow the hair to fully hydrate. The DSC was programmed to first heat each sample to 100 °C for 3 min and then to warm them further from 100 to 180 °C at a constant rate of 5 °C min'.

**Table 2: Mean denaturation temperatures and change in denaturation temperature for treatment with Composition 1 in accordance with the invention and Comparative Composition A.**

| **Treatment** | **Mean Denaturation Temperature (°C)** | **Standard Deviation (+/-)** |
|---|---|---|
| Virgin Hair | 147.17 | 0.33 |
| Double bleached hair | 142.58 | 0.11 |
| Double bleached hair + Composition 1 | 148.88 | 0.61 |
| Double bleached hair + Composition A | 142.58 | 0.11 |

It will be seen that hair treated with Shampoo Composition A has low denaturation temperature due to the nature of bleached hair. Shampoo Composition 1, in accordance with the invention, increases the denaturation temperature of bleached hair to higher that of virgin hair.

## Claims

1. A method of repairing damaged internal protein of hair by increasing the denaturation temperature of the internal protein, comprising the step of applying a shampoo composition to the hair, wherein the composition comprises a soluble zinc compound selected from zinc sulphate, zinc chloride, zinc gluconate, zinc acetate, zinc citrate and mixtures thereof and a cleansing surfactant selected from anionic surfactant and mixtures thereof.; and wherein the composition has a pH of from 3 to 5, and
leaving the composition on the hair for a maximum time period of 20 minutes and rinsing the composition from the hair.

2. A method according to claim 1 in which the zinc compound is zinc sulphate.

3. A method according to any preceding claim in which level of zinc compound in the total composition is from 0.005 to 10 wt%, more preferably from 0.01 to 5 wt%, most preferably from 0.05 to 2 wt% by weight of the total composition.

4. A method as claimed in any preceding claim, wherein the cause of the damage is selected from mechanical means, chemical means and environmental means.

5. Method as claimed in claim 4, wherein the damage is selected from lightening, chemical straightening, colouring, heat styling, and mixtures thereof.

6. A method according to any preceding claim wherein the maximum time period is of 5 minutes.

7. Use of a composition as defined in any of claims 1-3, to repair damage to internal hair protein.

## Patentansprüche

1. Verfahren zum Reparieren des inneren Proteins von geschädigtem Haar durch Anheben der Denaturierungstemperatur des inneren Proteins, umfassend den Schritt des Auftragens einer Shampoo-Zusammensetzung auf das Haar, wobei die Zusammensetzung eine lösliche Zinkverbindung, ausgewählt aus Zinksulfat, Zinkchlorid, Zinkgluconat, Zinkacetat, Zinkcitrat und Mischungen davon, und ein Reinigungstensid, ausgewählt aus anionischem Tensid und Mischungen davon, umfasst, und wobei die Zusammensetzung einen pH-Wert von 3 bis 5 aufweist, und
Belassen der Zusammensetzung auf dem Haar für eine maximale Zeitdauer von 20 Minuten und Abspülen der Zusammensetzung von dem Haar.

2. Verfahren nach Anspruch 1, wobei die Zinkverbindung Zinksulfat ist.

3. Verfahren nach irgendeinem vorhergehenden Anspruch, wobei der Gehalt an Zinkverbindung in der Gesamtzusammensetzung 0,005 bis 10 Gew.-%, bevorzugter 0,01 bis 5 Gew.-%, höchst bevorzugt 0,05 bis 2 Gew.-%, bezogen auf das Gewicht der Gesamtzusammensetzung, beträgt.

4. Verfahren, wie in irgendeinem vorhergehenden Anspruch beansprucht, wobei die Ursache des Schadens aus mechanischen Mitteln, chemischen Mitteln und Umweltmitteln ausgewählt ist.

5. Verfahren, wie in Anspruch 4 beansprucht, wobei der Schaden aus Aufhellen, chemischem Glätten, Färben, Hitzestyling und Mischungen davon ausgewählt ist.

6. Verfahren nach irgendeinem vorhergehenden Anspruch, wobei die maximale Zeitdauer 5 Minuten beträgt.

7. Verwendung einer Zusammensetzung, wie in irgendeinem der Ansprüche 1-3 definiert, zum Reparieren des Schadens von innerem Haarprotein.

## Revendications

1. Procédé de réparation de protéine de cheveux interne détériorée en augmentant la température de dénaturation de la protéine interne, comprenant l'étape consistant à appliquer une composition de shampooing aux cheveux, dans lequel la composition comprend un composé de zinc soluble choisi parmi le sulfate de zinc, chlorure de zinc, gluconate de zinc, acétate de zinc, citrate de zinc et des mélanges de ceux-ci et un tensioactif nettoyant choisi parmi un tensioactif anionique et des mélanges de ceux-ci ; et dans lequel la composition présente un pH de 3 à 5, et à
laisser la composition sur les cheveux sur une période maximale de 20 minutes et rincer la composition des cheveux.

2. Procédé selon la revendication 1, dans lequel le composé de zinc est le sulfate de zinc.

3. Procédé selon l'une quelconque des revendications précédentes, dans lequel la teneur en composé de zinc dans la composition totale est de 0,005 à 10 % en masse, encore mieux de 0,01 à 5 % en masse, bien mieux encore de 0,05 à 2 % en masse de la composition totale.

4. Procédé selon l'une quelconque des revendications précédentes, dans lequel la cause de la détérioration est choisie parmi un moyen mécanique, moyen chimique et moyen environnemental.

5. Procédé selon la revendication 4, dans lequel la détérioration est choisie parmi un éclaircissement, un lissage chimique, une coloration, un coiffage à chaud, et des mélanges de ceux-ci.

6. Procédé selon l'une quelconque des revendications précédentes, dans lequel la période maximale est de 5 minutes.

7. Utilisation d'une composition selon l'une quelconque des revendications 1-3, pour réparer une détérioration de protéine de cheveux interne.
